# EUROPEAN PATENT SPECIFICATION

(11) **EP 1 946 728 B1**
(45) Date of publication and mention of the grant of the patent: **06.01.2010**
(21) Application number: 07001976.5
(22) Date of filing: 30.01.2007
(51) Int. Cl.: A61F 5/56

(54) **Apparatus for preventing sleeping respiratory obstruction**
Apparat zum Verhindern der Blockade des Atemsystems beim Schlafen
Appareil pour empêcher l'obstruction du système de voie respiratoire

(30) Priority: 17.01.2007 KR 20070005211
(43) Date of publication of application: 23.07.2008
(73) Proprietor: Bio Sleep Med Co., Ltd., Seongbuk-gu Seoul (KR)
(72) Inventor: Hong, Junghwa, Dongan-gu Anyang-si Gyeonggi-do (KR); Shin, Chol, Seocho-gu Seoul (KR)
(74) Representative: Samson & Partner

(56) References cited:
- EP-A- 1 254 648
- DE-A1- 19 535 232
- DE-B- 1 198 005
- JP-A- 11 076 318
- JP-A- 2005 058 388
- JP-A- 2005 261 682
- US-A- 5 265 624

## Description

The present invention relates to an apparatus for preventing a sleeping respiratory obstruction; and, more particularly, to an apparatus for preventing a sleeping respiratory obstruction, which is capable of unaffectedly changing a sleep posture to stop a snoring or a sleep apnea without awakening the sleeper and capable of making such a posture change accurately even if a sleeper moves unconsciously by accurately determining whether or not the sleeper snores based on an ambient noise.

In general, a habitual snoring, an obstructive sleep apnea and an upper airway resistance syndrome classified as a sleep-disordered breathing are diseases in which the repetitive closure of the upper airway occurs during sleep. Such diseases hinder sound sleep by deteriorating sleep efficiency at night and especially decrease a blood oxygen saturation rate [see Chrokroverty S. (1994) Sleep Disorder Medicine. Butterworth-Heinemann].

The sleep-disordered breathing causes a daytime drowsiness, a deteriorated power of concentration, a memory loss, a decreased learning ability, a chronic fatigue and the like. Further, the sleep-disordered breathing leads to accidents at construction sites and workplaces and traffic accidents caused by a drowsy driving, thereby inflicting social and economical damages.

There have recently been several reports on a close relationship between the sleep-disordered breathing and the occurrence of obesity, high blood pressure, diabetes, dementia, cardiovascular diseases, cardiac paralysis, sexual function decline, cerebrovascular diseases, paralysis and metabolic syndrome [see Prospective study of the association between sleep-disordered breathing and hypertension. N Engl Med 2000; 342: 1378-1384].

An upper airway closure of a human body like this will be explained in the following with reference to the accompanying drawings.

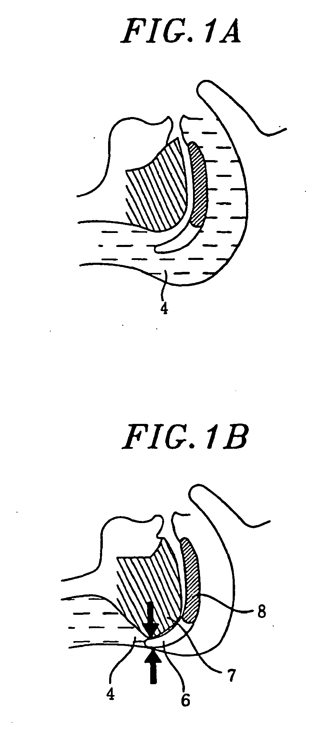

As shown in Fig. 1A, an upper airway 4 for introducing air into a bronchus and a lung (all not shown) is sufficiently secured in a normal state. However, referring to an obstructive sleep apnea state illustrated in Fig. 1B, a soft tissue 6 extended from a back part of a palate 8 is pressed by a self-weight and a weight of a tongue 7, thereby closing the upper airway 4.

Sleeping in a supine position worsens the closure of the upper airway 4. Further, such closure of the upper airway 4 leads to an obstructive sleep apnea in which breathing stops or is disrupted during sleep, and snoring occurs when the upper airway 4 is partially closed during sleep.

Various methods have been attempted to treat the snoring or the sleep apnea.

In general, there have been attempts of treating the sleep-disordered breathing by use of a functional pillow sheet during sleep, and there have been developed pillow sheets which prevent the sleep-disordered breathing through a posture correction by mainly elevating a lateral position or a head position.

Recently, a memory foam pillow sheet made of polymer foam developed by NASA has been commercially sold while claming as the pillow sheet for preventing a sleep-disordered breathing. Although such a memory foam pillow sheet is ergonomically designed to absorb load and shock transmitted to a human body and return to an original shape after the load is released, it does not bring a significant improvement for the treatment of a snoring or an sleep apnea.

Many apparatuses and methods have been developed for solving such problems, and this will be explained with reference to the accompanying drawings in the following.

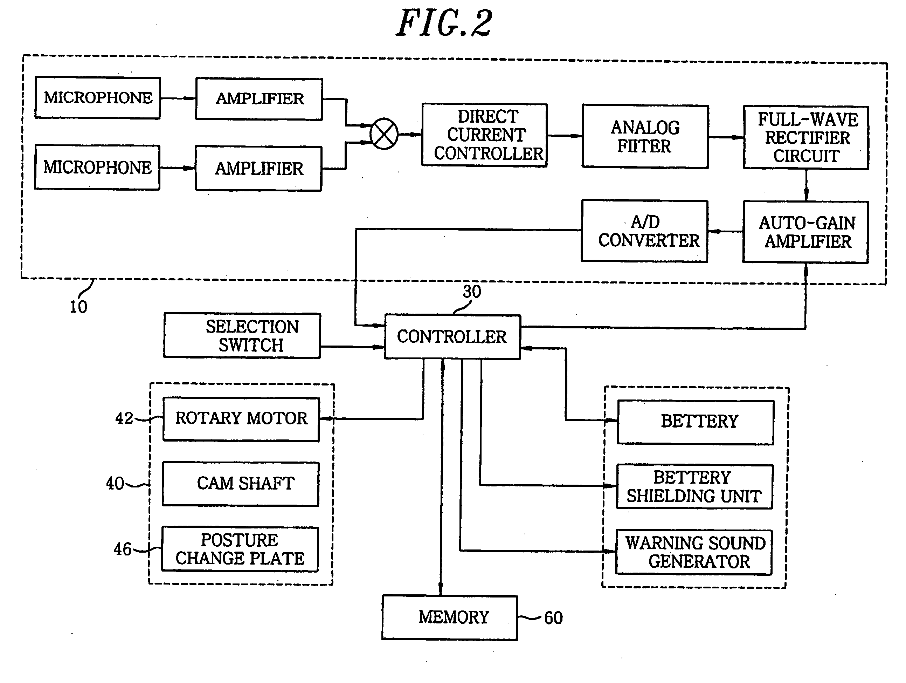

In accordance with an embodiment of a prior art, a utility model No. 20-0236225 which is filed with and registered in the Korean Intellectual Property Office, as shown in Fig. 2 is an "apparatus of driving a snoring prevention pillow sheet by sensing snoring." The snoring prevention apparatus includes a snoring signal sensing unit 10 which inputs snoring and various other noises and outputs a desired snoring signal; a controller 30 which analyzes the signal applied from the snoring signal sensing unit 10 and starts a rotary motor driver 40 if the applied signal is confirmed as a snoring signal; a memory which stores the signal applied from the controller 30 or outputs to the controller 30; and a rotary memory driver 40 where a rotary motor 42 is driven by the signal applied from the controller 30 to drive a posture change plate 46, thereby changing the shape of a pillow sheet. The controller 30 processes the snoring signal and the noise signal applied from the snoring signal sensing unit 10 to obtain a final data and calculate a time duration when the final data continuously has a value which is larger than a threshold value. The controller 30 calculates the number of maximum points which exist for the duration if the duration is higher when compared with a set-time. The controller 30 determines the signal as the snoring signal if the number of maximum points is higher than a set value for maximum points. Further, the controller 30 applies drive signals to the rotary motor driver 40 if the snoring signal is sensed as many times as the number of the set value to drive the rotary motor 42.

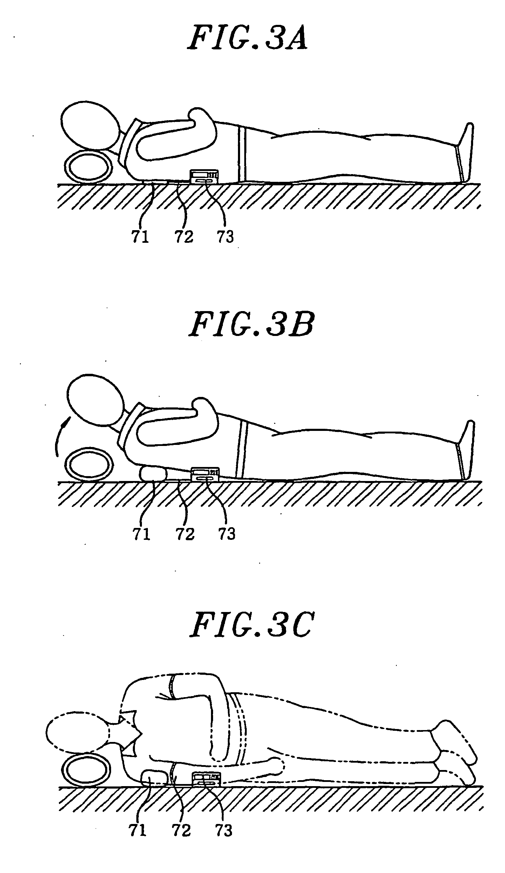

In accordance with another embodiment of the prior art, a utility model No. 20-0395890, which is filed for and registered in the Korean Intellectual Property Office, as shown in Figs. 3A to 3C is an "apparatus for reducing snoring." The snoring prevention apparatus includes an air pad 71 which detects the breath of a sleeper and is expanded by the air injected thereinto when snoring is sensed, thereby correcting the sleep posture of the sleeper; an air tube 72 which is combined with the air pad 71 to transmit the detected breathing to the next stage and to act as a passage of injecting and discharging air; a sensor for converting the breathing transmitted to the air tube 72 into an electrical signal; a preamplifier which pre-amplifies the breathing sense signal outputted from the sensor; a band pass filter unit which filters the breathing sense signal outputted from the preamplifier for each band and outputs it as a heart beat/ breathing/ snoring determinging signal; a function input unit for inputting function and mode conversion signals; a central processing unit 73 which determines whether or not there is snoring based on the signal obtained by the preamplifier and the band pass filter unit in response to the signal inputted from the function input unit, which controls to display a breathing state, generates an air injection signal for expanding the air pad if it is determined as snoring, and generates a control signal to discharge the air injected into the air pad after the set time if no snoring is sensed after the air pad is expanded; a display which displays on a screen the breathing state, the heart beat status and the like of the sleeper in accordance with the state display control signal transmitted from the central processing unit 73; a driver for outputting a light emitting diode control signal, the air injection control signal and a discharge control signal, which are outputted from the central processing unit, as each corresponding drive signal for each of the air injection control and the discharge control signal; a light emitting diode (LED) for visually displaying whether or not power is supplied and a function operation state in accordance with a light emitting diode drive signal outputted from the driver; a motor for driving a pump which injects air to the air pad 71 in accordance with the air injection signal outputted from the driver; and a discharge solenoid which opens and closes a discharge valve in accordance with the discharge control signal outputted from the driver.

In this way, the snoring prevention apparatus in accordance with the embodiment of the prior art in Fig. 2 automatically drives a snoring prevention pillow sheet to incline the sleeper to one side if snoring is sensed and determined, thereby enabling the sleeper not to snore. However, the snoring prevention apparatus according to the embodiment of the prior art cannot return to the original state from a state in which the snoring prevention pillow is inclined after sensing a snoring, unless the user manually resets the apparatus. There is a problem in operating such an apparatus reliably in that it becomes hard to prevent snoring if the sleeper moves away from the pillow sheet through movement or position change during sleep. Further, the sleeper's sound sleep might be disturbed because noise is generated due to a mechanical friction between a cam shaft and a plate.

Further, the snoring prevention apparatus according to another embodiment of the prior art in Figs. 3A to 3C induces posture change by use of the air pad 71, but is not an active type and could awake the sleeper by disturbing the sound sleep of the sleeper because it is a type which encourages to change himself/herself his/her postures. There also lies a problem in operating such an apparatus reliably because, in case that the sleeper moves away from the expansion portion of the air pad 71 when the sleeper moves or changes his position, even such a posture change cannot be induced.

JP 2005 261682 A discloses a snore suppressing apparatus comprising a pillow part wherein a pair of left and right air bags are accommodated, a snore sensor and means to supply air to the airbags when snore of the sleeping person is detected. This document discloses the features of the preamble of claim 1. JP 2005 058388 A describes an snoring prevention device comprising an air bag mounted between a person sleeping in supine position and bedding. An air flow rate to be supplied to the air bag when the snoring is sensed by a snoring sensor is varied by a flow rate adjusting valve. DE 195 35 232 A1 discloses an adjustable support for a pillow having an acoustic detector which when detecting snoring noises drives a means for elevating either side of the support. EP 1 254 648 A1 describes a device for preventing snoring wherein a pillow may be moved by a control unit to change the position of the sleeping person when a snoring noise is detected. US 5,265,624 A discloses a system for the treatment of obstructive sleep apnoea packaged in a collar assembly with remote electrodes which can be worn by a patient without any special preparation. Radio frequency signals are sent from the collar assembly to the remote electrodes to provide the therapy upon detection of an apnoea event. DE 11 98 005 B relates to a device for preventing snoring wherein a sleeper is retained at a vertical or inclined wall by a fastening means.

It is, therefore, a primary object of the present invention to provide to an apparatus for preventing a sleeping respiratory obstruction which is capable of unaffectedly changing a sleep posture to stop a snoring or a sleep apnea without awakening the sleeper and capable of making such a posture change accurately even if the sleeper is made to move unconsciously by accurately determining whether or not the sleeper snores or undergoes a sleep apnea based on an ambient noise, thereby enabling the sleeper to carry on a sound sleep and preventing a disordered breathing such as an obstructive sleep apnea.

In accordance with an aspect of the present invention, there is provided a sleeping respiratory obstruction prevention apparatus for preventing snoring or sleep apneas, including:
a human body wearable unit to be worn by a sleeper;
air chambers provided at a rear of the human body wearable unit, the air chambers being capable of being shrunk and expanded;
an air supply unit for supplying air to the air chambers;
a pressure detecting unit for measuring each pressure of the air chambers and outputting each pressure as an electrical signal; and
a controller for receiving the signal outputted from the pressure detecting unit to supply the air to the air chambers by using the air supply unit when a snoring or a sleep apnea of the sleeper is detected in order to change a posture of the sleeper.

There is also described a sleeping respiratory obstruction prevention method for preventing snoring or sleep apneas in which a sleeper wears a human body wearable unit having a shrinkable and expandable air chambers provided thereat at a rear thereof, the method including the steps of:
supplying air for a pressure measurement to the air chambers;
measuring the pressure of the air chambers pressurized by a weight of the sleeper;
extracting a bio-signal from the pressure;
determining whether or not a snoring or a sleep apnea is detected; and
supplying an air to the air chambers to expand the air chambers if the snoring or the sleep apnea is detected in the bio-signal, in order to change a posture of the sleeper.

The above and other objects and features of the present invention will become apparent from the following description of embodiments, given in conjunction with the accompanying drawings, in which:
Figs. 1A and 1B are drawings explaining a closure of an upper airway of human body;
Fig. 2 is a drawing illustrating a main part of a conventional snoring prevention apparatus;
Figs. 3A to 3C are drawings illustrating another conventional snoring prevention apparatus;
Figs. 4A and 4B show a front and a rear view of a sleeping respiratory obstruction prevention apparatus in accordance with an embodiment of the present invention;
Fig. 5 depicts a configuration diagram of the sleeping respiratory obstruction prevention apparatus in accordance with the embodiment of the present invention;
Fig. 6 is a flow chart illustrating a sleeping respiratory obstruction prevention method according to the present invention; and
Figs. 7A to 7C are drawings for explaining the operation of the sleeping respiratory obstruction prevention apparatus according to the present invention.

Hereinafter, embodiments of the present invention will be described in detail with reference to the accompanying drawings.

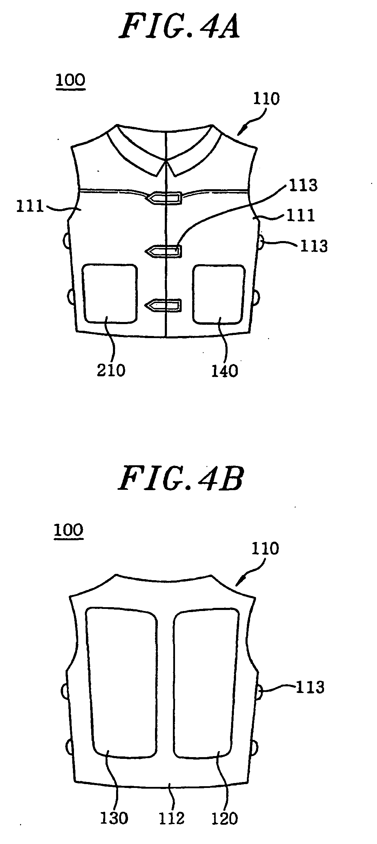

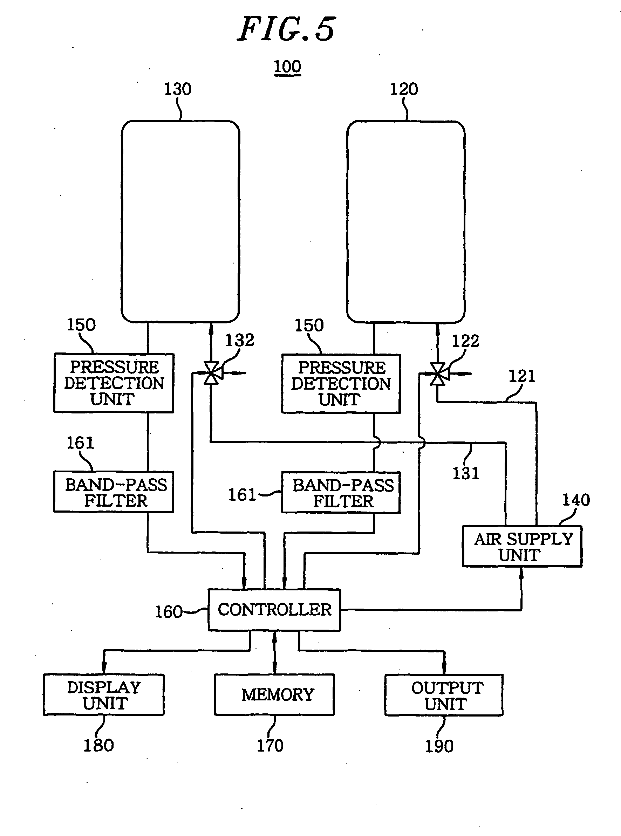

Figs. 4A and 4B show a front and a rear view of a sleeping respiratory obstruction prevention apparatus in accordance with an embodiment of the present invention, and Fig. 5 shows a configuration diagram of the sleeping respiratory obstruction prevention apparatus in accordance with the embodiment of the present invention. As shown above, the sleeping respiratory obstruction prevention apparatus 100 of the present invention includes a human body wearable unit 110 which to be worn by a sleeper; air chambers 120, 130 provided at the rear of the human body wearable unit 110; an air supply unit 140 for supplying air to the air chambers 120, 130; a pressure detecting unit 150 for measuring the pressure of the air chambers 120, 130; and a controller 160 which receives the signal outputted from the pressure detecting unit 150 and controls the air supply unit 140.

The human body wearable unit 110 to be worn by the sleeper adopts a design of a wearable unit while considering wearability and usability from a number of different design angles. Further, a functional structure and a suitable material selection are essential in such a wearable design. In particular, a material for regulating human body from harmful factors such as pleasantness, stability, electromagnetic wave shielding function, static electricity prevention function, insulation function and the like, are controlled can be selected.

In order to carry out a temperature control function, the human body wearable unit 110 not only discharges the sweat excreted during sleep through the material, but is also configured to have air holes in all directions of the body or in the armpit region where sweat can come out easily, so that the heat or humidity within the clothes can be discharged quickly, thereby improving the wear comfort. And, it is necessary to consider a sewing method of enabling for minimizing the friction between the material and the human body. In this regard, the human body wearable unit 110 may be made of health oriented materials (e.g., chitosan fiber, silver fiber, bamboo fiber or the like, hi-tech materials such as aqua-trans, cool max mesh or the like), or environment friendly materials (e.g., organic cotton, tencel, natural mineral ion health fiber or the like).

The human body wearable unit 110, though not shown in drawings, can be configured in a winter jacket or a jumper form. Considering that it is to be worn during sleep, it may be configured in a vest shape as shown in Figs. 4A and 4B. Further, the human body wearable unit 110 may be formed in a sleeveless vest form to make it better in permeability compared to a vest having sleeves, which in turn restrain factors increasing body temperature during sleep.

The human body wearable unit 110 can be made in various vest shapes such as a shape of having only front adjustment, a shape of having front adjustment and side open, a shape of having neither adjustment nor open, or the like. However, the human body wearable unit 110 might be made in a shape of having front adjustment and side open in this embodiment, and includes a pair of front portions 111 which covers the upper front surface of the sleeper and a rear portion 112 which covers the upper rear surface of the sleeper. The front adjustment between the front portions 111 and the side open between the front part 111 and the rear portion 112 are connected to be separable by easy-to-use fasteners 113 with which gap adjustment is easy.

The fastener 113 of the human wearable unit 110 is only an example, and various other combining and disjoining units other than the fastener such as zippers, rubber bands, snap buttons and the like can be used in conjunction with the same or a different kind, in order to connect the front adjustment and the side open.

Since the human body wearable unit 110 can be worn even when the front adjustment and the side open are opened, wearing and taking off the unit 110 are made easy even for patients having restricted body movement. Further, air ventilation is possible through the front adjustment and side open, thus the factors that increase the body temperature during sleep are greatly restrained. In addition, the human body wearable unit 110 can be worn for any body sizes and possibly minimize pressure working against body during sleep, which in turn aids the sleeper to have a sound sleep.

The air chambers 120, 130 are provided at the rear of the human body wearable unit 110, i.e., at the rear portion 112 and they can be shrunk or expanded. The air chambers 120, 130 are expanded by air supplied from the air supply unit 140 and are made in a structure or/and of a material which can be shrunk and expanded. In such a structure, the air chambers 120, 130 may include a part which can be sheared or darted. Further, for such a material, the air chambers 120, 130 might be employ, e.g., synthetic resin, natural rubber, artificial rubber, fiber and the like, which can be expanded or shrunk. The air chambers 120, 130 can have a sufficient volume to change the posture of the sleeper, e.g., from a supine position to a lateral position.

The air chambers 120, 130 are positioned to incline the sleeper to one side at the rear of the human body wearable unit 110, so as to easily and naturally change the sleeper posture from the supine position to the lateral position when expanded. Here, the air chambers 120, 130 can be provide in a pair as illustrated in the embodiment of the present invention so as to alternately change the posture of the sleeper from the supine position to the lateral position of left side or right side.

The air supply unit 140 selectively supplies air to the air chambers 120, 130 via air supply lines 121, 131 in accordance with the control signal of the controller 160. In this regard, a compressed gas can be used, and an air pump may be used to supply air through pumping.

The pressure detecting unit 150 includes a pressure measuring sensor and is installed on a route (not shown) connected to the air chambers 120, 130. The pressure detecting unit 150 measures a pressure change of the air chambers 120, 130, the pressure change being caused by the heart beat, the breathing and the like of the sleeper. It also measures the pressure of the air chambers pressurized by, for example, the weight of the sleeper, and outputs thus measured pressure value as an electrical signal to the controller 160. Meanwhile, to measure the pressure with the pressure detecting unit 150, the air chambers 120, 130 are required to be filled with air, thereby exerting an enough volume required for a pressure measurement. Here, the filled air may be an amount with which the sleeper does not feel discomfort when lying down in the supine position.

The controller 160 receives the signal transmitted from the pressure detecting unit 150. Further, upon sensing a snoring or a sleep apnea, the air supply unit 140 is configured to supply air to the air chamber in order to change the posture of the sleeper, e.g., from the supine position to the lateral position, thereby opening the upper airway of the sleeper, which in turn corrects the disordered breathing of the sleeper. In case that the air chambers 120, 130 are provided in a pair as illustrated in the embodiment of the present invention, the controller 160 receives the signal from the pressure detecting unit 150 measuring each pressure of the air chambers 120, 130, and alternately expands the air chambers 120, 130 in order to put the sleeper in the lateral position facing a direction different from a previous position each time a snoring or a sleep apnea is detected.

The controller 160 converts the signal transmitted from the pressure detecting unit 150 into a plurality of frequencies by using a plurality of band pass filters 161 and calculates any one of the number of breathings, the number of heart beats, the number of snores, the number of apneas and the like of the sleeper or the combination thereof from each of frequency bands.

For example, the controller 160 receives the changed pressure of the air chambers 120, 130 as an electrical signal from the pressure detecting unit 140. Further, the controller 160 calculates the number of breathings and the number of heart beats by analyzing waveform cycles thereof in the 0.1-0.5Hz band and the 0.7-2.0Hz band, which are obtained by the filtering of the band pass filter 161, and calculates the number of snores or sleep apnea based on the number of the waveforms which exist in a band of over 50Hz. Such a frequency band can vary in numerical value depending on a physical condition of the sleeper, a clinical trial method and the like. Particularly, the presence or the absence of the snoring or the sleep apnea can be determined by observing a frequency characteristic which is based on the result obtained in the clinical trial by use of a STFT (Short Time Fourier Transformation) method. Further, a sleep apnea in general refers to a situation where a person does not breathe for longer than about 10 seconds or so and such a situation occurs 5 or more times in an hour. Since the embodiment of the present invention is to prevent sleep apneas by encouraging a posture change when such a sleep apnea is occurred, the sleep apnea is determined to be present when no breathing is detected in a time period of 10 seconds.

Meanwhile, in order to store any one of the number of breathings, the number of heart beats, the number of snores and the number of apneas calculated by the controller 160 or the combination thereof as data, a memory 170 is provided. Further, a display unit 180 can be provided to externally display any one of the maximum number of breathings per minute, the minimum number of breathings per minute, the average number of breathings per minute, the cumulative number of snoring or the combination thereof based on a selection signal from the data stored at the memory part 170, e.g., a manipulation signal from an input unit given by a user. Further, an output unit 190 such as a USB (Universal Serial Bus) port and the like is provided to transmit the data stored at the memory 170 to an external device, e.g., a personal computer, a medical device, an analytical device and the like.

The controller 160 can control the air supply unit 140 to directly shrink as well as expand the air chambers 120, 130. For example, if the air supply unit 140 is an air pump, the shrinkage and the expansion are achieved through a forward rotation and a reverse rotation by the pump. Further, although it is not limited thereto, the three-way valves 122, 132, as in the embodiment of the present invention, controlled by the controller 160 are installed on a route, i.e., the air supply line 121, 131, respectively, through which the air is supplied from the air supply unit 140, so that the expanded air chambers 120, 130 can discharge the air for shrinkage.

The three-way valves 122, 132 may be solenoid valves which can be operated by control signals from the controller 160, for example.

On the other hand, the air supply unit 140, the controller 160, the memory 170, the display unit 180 and the like can be installed to be separated from the human body wearable unit 110, but can also be separately attached thereto for the sake of a convenient use and in order to minimize the interference with the sleeper. Here, the controller 160, the memory 170, the display unit 180can be installed within a control box 210 (shown in Fig. 4A) of which the outer surface has a plurality of manipulation buttons (not shown) and stuck to the human body wearable unit 110, and the power supplied to the air supply unit 140, the controller 160 and the like is provided by an easy-to-use rechargeable battery.

The operation of the sleeping respiratory obstruction prevention apparatus made in such a structure will be superseded with a detail explanation for a sleeping respiratory obstruction prevention method

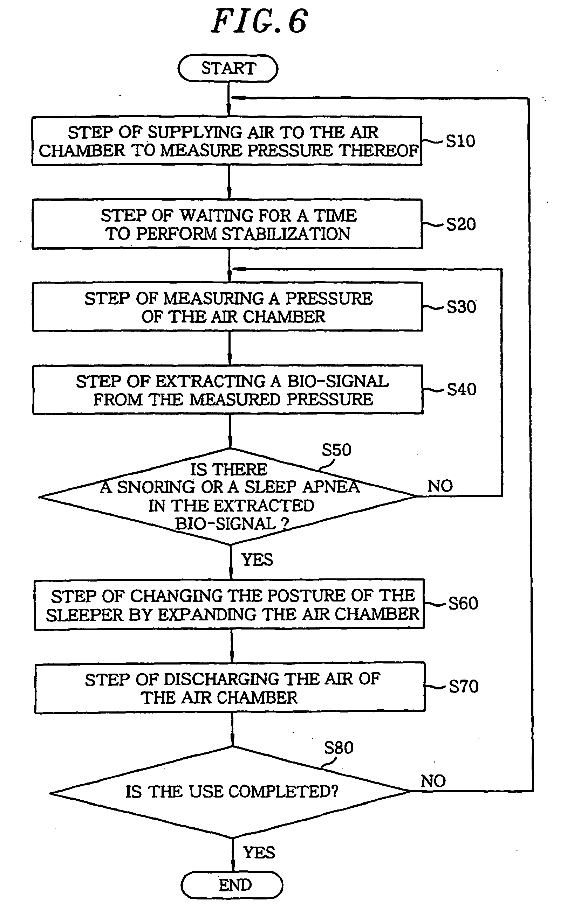

Fig. 6 is a flow chart illustrating a sleeping respiratory obstruction prevention method. As shown in the drawings, the sleeping respiratory obstruction prevention method of the present invention is for preventing the snoring or the sleep apnea of the sleeper who wears the human body wearable unit 110 where there is provided the shrinkable and expandable air chamber at the rear thereof. The sleeping respiratory obstruction prevention method includes the steps of: supplying air for measuring pressure to the air chambers (S10); measuring the pressure of the air chambers (S30); extracting a bio-signal from thus measured pressure (S40); determining whether or not the snoring or the sleep apnea is revealed in the bio-signal (S50); and changing the posture of the sleeper if the snoring or the sleep apnea is detected (S60).

In the step of supplying the air for measuring pressure to the air chamber (S10), if the device is configured to be started when the sleeper lies down straight while wearing the human body wearable unit 110, the air with which the pressure of the air chambers 120, 130 can be measured is supplied to the extent that the sleeper does not feel discomfort while lying down in a supine position.

Meanwhile, in order to accurately measure the pressure of the air chambers 120, 130 after the step of supplying the air for measuring pressure, a time delay is added during which no pressure is measured for a specific time, e.g., 15-25 seconds or preferably around 20 seconds to stabilize the air chambers 120, 130.

In the step of measuring the pressure of the air chamber (S30), the pressures of the air chambers 120, 130 pressurized by the weight of the sleeper are measured by the pressure detecting unit 150 after the step of stabilizing the air chamber (S20). Here, the duration of measurement is about 5 to 15 seconds, preferably around 10 seconds. The pressure detecting unit 150 detects a pressure change within the air chambers 120, 130 based on the heart beat, the breathing or the like of the sleeper and outputs the pressure change as an electrical signal to the controller 160.

In the step of extracting the bio-signal from the measured pressure (S40), the electrical signal for the input value measured by the pressure detecting unit 150 is filtered by one or a plurality of band pass filters 161 for each frequency band. Further, the number of breathings, the number of heart beats, the number of snores and the number of apneas are calculated or the combination thereof is calculated based on each frequency band, as described above. Here, in case of snoring or sleep apneas, the presence or absence thereof can be determined by observing a frequency characteristic based on the result obtained in the clinical trial by use of a STFT (Short Time Fourier Transformation) method. Further, a sleep apnea in general refers to a situation where a person does not breathe for longer than about 10 seconds or so and such a situation occurs 5 or more times in an hour. Since the embodiment of the present invention is to prevent sleep apneas by encouraging a posture change when such a sleep apnea is occurred, the sleep apnea is determined to be present when no breathing is detected in a time period of 10 seconds.

Meanwhile, the extracted bio-signal can be externally displayed as a maximum value, a minimum value, an average and the like thereof via the display unit 180, and the data can be transmitted to the output unit 190 through an external device such as a personal computer, the diagnostic device and the like.

In the step of determining whether or not the snoring or the sleep apnea is detected in the bio-signal (S50), the presence of snoring or the sleep apnea is determined based on one or a plurality of data obtained form the sleeper. The step of changing the posture of the sleeper (S60) is carried out upon detecting the snoring or the sleep apnea.

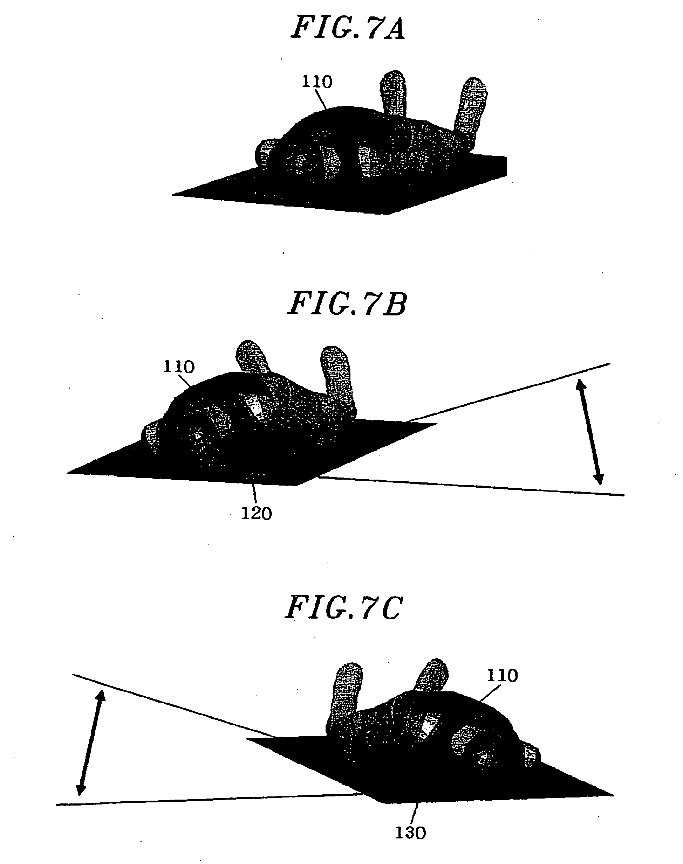

When the snoring or the sleep apnea is detected, the air supply unit 140 starts supplying air to the air chambers 120, 130, thereby expanding the air chambers 120, 130. Here, in case that the air chambers 120, 130 are provided at a left side and a right side of the rear of the human body wearable unit 110, respectively, as in this embodiment, the air is first supplied to any one 120 of the air chambers 120, 130, thereby changing the posture of the sleeper from the supine position (shown in Fig. 7A) to the lateral position (shown in Fig. 7B), which in turn opens the upper airway correcting the disordered breathing of the sleeper.

In the step of changing the posture of the sleeper (S60), when a fixed time, e.g., between 5 and 10 minutes, elapses after the posture of the sleeper is changed, the air of the air chamber 120 is discharged so that the posture of the sleeper can return to the normal state (S70). Here, the air discharge of the air chamber 120 can be performed by the reverse operation of the air supply unit 140. However, in this embodiment, the discharge can be made through the three-way valve 122 which operates based on the control signal from the controller 160, in order to naturally bring the sleeper to the normal position through a smooth air discharge.

Meanwhile, if the sleeper returns to the supine position by the air discharge of the air chamber (S70), as shown in Fig. 7A, whether or not the usage is completed is determined (S80), and if it is still in use, the steps following the step of measuring the sound generated by the sleeper (S10) are performed. Further, when the snoring or the sleep apnea is detected, the air is supplied to the air chamber 130 different from the previous one, thereby changing the posture to the lateral position of the opposite direction, as shown in Fig. 7C. That is to say, in the step of changing the posture of the sleeper (S60), the air chambers 120, 130 located at the a left and a right side of the rear of the human body wearable unit 110, respectively, are alternately expanded to achieve a different lateral position for the sleeper each time the snoring or the sleep apnea is detected or the sleep apnea.

In the step of changing the posture of the sleeper (S60), in case a single air chamber is provided to incline the sleeper to one side at the rear of the human body wearable unit 110, the sleeper is changed from the supine position to the lateral position by expanding the single air chamber.

The pressure of snoring or sleep apnea by the sleeper can preferably be accurately determined by the pressure change within the air chambers 120, 130 pressurized by the weight, heart beat, breathing and the like of the sleeper. Further, when the snoring or the sleep apnea is detected, the sleep posture is naturally changed from the supine position to the lateral position by selectively expanding the air chambers 120, 130 without awakening the sleeper so as to stop the snoring or the sleep apnea, thereby keeping the sleeper to have a sound sleep and preventing the disordered breathing such as the snoring, the sleep apnea and the like.

Further, the human body wearable unit 110 having the air chambers 120, 130 provided at the rear thereof is worn by the sleeper, thus the operation capable of changing the posture of the sleeper for preventing the snoring or the sleep apnea is highly reliable even though there is a change in location and posture during sleep. Further, the expanded air chambers 120, 130 are shrunk to its original state, to thereby enable the sleeper to return to the comfortable posture. In addition, the air chambers 120, 130 are operated by the supply of the air, thereby restraining the noise generated upon the operation thereof to minimize the sleep disturbance.

As described above, an apparatus for preventing a sleeping respiratory obstruction in accordance with the embodiment of the present invention can accurately determine whether or not the sleeper snores or under goes the sleep apnea through the pressure change within the air chambers pressurized by the sleeper, and can naturally change the sleep posture by expanding the air chambers without awakening the sleeper so as to stop snoring or sleep apneas when the sleeper snores or undergoes a sleep apnea. Thus, the sleeper can be kept to have a sound sleep, and the disordered breathing such as the snoring, the sleep apnea and the like might be prevented. Further, the human body wearable unit 110 having the air chambers provided at the rear thereof is worn by the sleeper, thus the operation for preventing the snoring or the sleep apnea is highly reliable even though the position or the posture is changed during sleep. Further, the expanded air chamber is shrunk to the original state, thus the sleeper can return to a comfortable posture while the noise generated upon the operation is restrained to minimize the sleep disturbance.

While the invention has been shown and described with respect to the preferred embodiments, it will be understood by those skilled in the art that various changes and modification may be made without departing from the scope of the invention as defined in the following claims.

## Claims

1. A sleeping respiratory obstruction prevention apparatus (100) for preventing snoring or sleep apneas, comprising:
air chambers (120,130) provided at a rear of the human body wearable unit, the air chambers being capable of being shrunk and expanded;
an air supply unit (140) for supplying air to the air chambers; **characterized in** further comprising:
a human body wearable unit (110) to be worn by a sleeper;
a pressure detecting unit (150) for measuring each pressure of the air chambers and outputting each pressure as an electrical signal; and
a controller (160) for receiving the signal outputted from the pressure detecting unit (150) to supply the air to the air chambers (120,130) by using the air supply unit (140) when a snoring or a sleep apnea of the sleeper is detected in order to change a posture of the sleeper.

2. The sleeping respiratory obstruction prevention apparatus of claim 1, wherein the human body wearable unit is formed in a vest shape.

3. The sleeping respiratory obstruction prevention apparatus of claim 1, wherein one of the air chambers is positioned to incline the sleeper to one side at the rear of the human body wearable unit, in order to change the posture of the sleeper from a supine position to a lateral position upon expansion.

4. The sleeping respiratory obstruction prevention apparatus of claim 1, wherein the air chambers include three-way valves controlled by the controller and installed on a route through which the air is supplied to discharge the air.

5. The sleeping respiratory obstruction prevention apparatus of claim 1 or 4, wherein two of the air chambers are provided at a left and a right side of the rear of the human body wearable unit, respectively, and
the controller receives a signal from the pressure detecting unit which measures each pressure of the air chambers and alternately expands the air chambers so as to put the sleeper in a lateral position facing a direction different from a previous position each time the snoring or the sleep apnea is detected.

6. The sleeping respiratory obstruction prevention apparatus of claim 1, further comprising:
a memory that filters the signal outputted from the pressure detecting unit and stores any one of the number of breathings, the number of heart beats, the number of snores and the number of apneas of the sleeper, which are calculated by the controller, or a combination thereof.

7. The sleeping respiratory obstruction prevention apparatus of claim 6, further comprising:
a display unit which externally displays any one of a maximum number of breathings per minute, a minimum number of breathings per minute, an average number of breathings per minute, a cumulative number of snoring and a cumulative number of apneas, or a combination thereof based on a selection signal from the data stored at the memory.

8. The sleeping respiratory obstruction prevention apparatus of claim 6, further comprising:
an output unit for transmitting the data stored at the memory to an external device.

## Patentansprüche

1. Vorrichtung (100) zum Verhindern der Blockade des Atemsystems beim Schlafen zum Verhindern von Schnarchen oder Schlafapnoen, welche umfasst:
Luftkammern (120, 130), die an einer Rückseite der vom menschlichen Körper tragbaren Einheit vorgesehen ist, wobei die Luftkammern geschrumpft und expandiert werden können;
eine Luftzuführeinheit (140) zum Zuführen von Luft den Luftkammern; **dadurch gekennzeichnet** sie ferner umfasst:
eine vom menschlichen Körper tragbare Einheit (110), die von einem Schläfer getragen wird;
eine Druckdetektiereinheit (150) zum Messen jedes Drucks der Luftkammern und Ausgeben jedes Drucks als ein elektrisches Signal; und
eine Steuereinheit (160) zum Empfangen des von der Druckdetektiereinheit (150) ausgegebenen Signals, um die Luft den Luftkammern (120, 130) zuzuführen durch Verwenden der Luftzuführeinheit (140), wenn ein Schnarchen oder eine Schlafapnoe des Schläfers detektiert wird, um eine Lage des Schläfers zu verändern.

2. Vorrichtung zum Verhindern der Blockade des Atemsystems gemäß Anspruch 1, wobei die vom menschlichen Körper tragbare Einheit in einer Westenform gebildet ist.

3. Vorrichtung zum Verhindern der Blockade des Atemsystems gemäß Anspruch 1, wobei eine der Luftkammern positioniert ist, um den Schläfer zu einer Seite an der Rückseite der vom menschlichen Körper tragbaren Einheit zu neigen, um die Lage des Schläfers von einer Rückenlage in eine Seitenlage beim Expandieren zu wechseln.

4. Vorrichtung zum Verhindern der Blockade des Atemsystems gemäß Anspruch 1, wobei die Luftkammern Drei-Wege-Ventile umfassen, die von der Steuereinheit gesteuert werden und auf einem Weg eingebaut sind, durch den die Luft zugeführt wird, um die Luft zu entlassen.

5. Vorrichtung zum Verhindern der Blockade des Atemsystems gemäß Anspruch 1 oder 4, wobei zwei der Luftkammern an einer linken bzw. einer rechten Seite der Rückseite der vom menschlichen Körper tragbaren Einheit vorgesehen sind, und
die Steuereinheit ein Signal aus der Druckdetektiereinheit empfängt, die jeden Druck der Luftkammern misst und abwechselnd die Luftkammern expandiert, um den Schläfer in eine Seitenposition zu legen, die in eine Richtung zeigt, die anders ist als eine vorherige Position, jedes Mal wenn das Schnarchen oder die Schlafapnoe detektiert wird.

6. Vorrichtung zum Verhindern der Blockade des Atemsystems gemäß Anspruch 1, welche ferner umfasst:
einen Speicher, der das aus der Druckdetektiereinheit ausgegebene Signal filtert und eines von: die Anzahl der Atemzüge, die Anzahl der Herzschläge, die Anzahl der Schnarcher und die Anzahl der Apnoen des Schläfers speichert, die von der Steuereinheit oder einer Kombination davon berechnet werden.

7. Vorrichtung zum Verhindern der Blockade des Atemsystems gemäß Anspruch 6, welche ferner umfasst:
eine Anzeigeeinheit, die nach außen eine maximale Anzahl von Atemzügen pro Minute, eine minimale Anzahl von Atemzügen pro Minuten, eine durchschnittliche Anzahl von Atemzügen pro Minute, eine kumulative Anzahl von Schnachern und eine kumulative Anzahl von Apnoen oder eine Kombination davon auf der Grundlage eines Auswahlsignals aus den im Speicher gespeicherten Daten anzeigt.

8. Vorrichtung zum Verhindern der Blockade des Atemsystems gemäß Anspruch 6, welche ferner umfasst:
eine Ausgabeeinheit zum Übertragen der im Speicher gespeicherten Daten zu einer externen Vorrichtung.

## Revendications

1. Appareil pour empêcher l'obstruction du système respiratoire pendant le sommeil (100) pour empêcher les ronflements ou l'apnée du sommeil, comprenant :
➢ des chambres à air (120, 130) prévues au niveau d'une partie arrière de l'unité pouvant être portée par le corps humain, les chambres à air pouvant se contracter et se dilater ;
➢ une unité d'alimentation d'air (140) pour alimenter de l'air aux chambres à air ; **caractérisé en ce qu'**il comprend en outre :
➢ une unité pouvant être portée par un corps humain (110) destinée à être portée par une personne qui dort ;
➢ une unité de détection de pression (150) pour mesurer chaque pression des chambres à air et reproduire chaque pression sous la forme d'un signal électrique ; et
➢ un contrôleur (160) pour recevoir le signal produit par l'unité de détection de pression (150) pour alimenter l'air aux chambres de pression (120, 130) en utilisant l'unité d'alimentation d'air (140) lorsqu'un ronflement ou une apnée du sommeil de la personne qui dort est détecté afin de changer une position de la personne qui dort.

2. Appareil pour empêcher l'obstruction du système respiratoire pendant le sommeil selon la revendication 1, dans lequel l'unité pouvant être portée par le corps humain a une forme de gilet.

3. Appareil pour empêcher l'obstruction du système respiratoire pendant le sommeil selon la revendication 1, dans lequel l'une des chambres à air est positionnée pour incliner la personne qui dort d'un côté à l'arrière de l'unité pouvant être portée par un corps humain, afin de changer la position de la personne qui dort d'une position étendue sur le dos' à une position latérale suite à l'expansion.

4. Appareil pour empêcher l'obstruction du système respiratoire pendant le sommeil selon la revendication 1, dans lequel les chambres à air comprennent des soupapes à trois voies contrôlées par le contrôleur et installées sur une voie à travers laquelle l'air est alimenté pour décharger l'air.

5. Appareil pour empêcher l'obstruction du système respiratoire pendant le sommeil selon la revendication 1 ou 4, dans lequel deux des chambres à air sont prévues sur un côté gauche et un côté droit de l'arrière de l'unité pouvant être portée par un corps humain, respectivement, et le contrôleur reçoit un signal de l'unité de détection de pression qui mesure chaque pression des chambres à air et expanse de manière alternée les chambres à air afin de placer la personne qui dort dans une position latérale orientée dans une direction différente d'une position précédente chaque fois que le ronflement ou l'apnée du sommeil est détecté.

6. Appareil pour empêcher l'obstruction du système respiratoire pendant le sommeil selon la revendication 1, comprenant en outre une mémoire qui filtre le signal produit par l'unité de détection de pression et stocke l'un quelconque parmi le nombre de respirations, le nombre de battements cardiaques, le nombre de ronflements et le nombre d'apnées de la personne qui dort qui sont calculés par le contrôleur ou leur combinaison.

7. Appareil pour empêcher l'obstruction du système respiratoire pendant le sommeil selon la revendication 6, comprenant en outre une unité d'affichage qui affiche extérieurement l'un quelconque parmi un nombre maximum de respirations par minute, un nombre minimum de respirations par minute, un nombre moyen de respirations par minute, un nombre cumulé de ronflements et un nombre cumulé d'apnées ou leur combinaison en fonction d'un signal de sélection provenant des données stockées dans la mémoire.

8. Appareil pour empêcher l'obstruction du système respiratoire pendant le sommeil selon la revendication 6, comprenant en outre une unité de sortie pour transmettre les données stockées dans la mémoire à un dispositif externe.
